# EUROPÄISCHE PATENTSCHRIFT

(11) **EP 1 286 976 B1**
(45) Veröffentlichungstag und Bekanntmachung des Hinweises auf die Patenterteilung: **09.03.2011**
(21) Anmeldenummer: 01947242.2
(22) Anmeldetag: 26.04.2001
(51) Int. Cl.: C07D 277/32

(54) **VERFAHREN ZUR HERSTELLUNG VON 2-CHLOR-5-CHLORMETHYL-1,3-THIAZOL**
METHOD FOR PRODUCING 2-CHLORO-5-CHLOROMETHYL-1,3-THIAZOLE
PROCEDE DE PRODUCTION DE 2-CHLORO-5-CHLOROMETHYL-1,3-THIAZOL

(30) Priorität: 23.05.2000 AT 8952000
(43) Veröffentlichungstag der Anmeldung: 05.03.2003
(73) Patentinhaber: Syngenta Participations AG, 4058 Basel (CH)
(72) Erfinder: KRICH, Sylvia, A-4203 Altenberg (AT); BURGER, Christian, A-4060 Leonding (AT); ALTREITER, Johann, A-4212 Neumarkt (AT); ZWÖLFER, Birgit, A-4850 Timelkam (AT)
(74) Vertreter: Waterman, John Richard
(86) Internationale Anmeldenummer: PCT/EP2001/004693
(87) Internationale Veröffentlichungsnummer: WO 2001/090089

(56) Entgegenhaltungen:
- EP-A- 0 260 560

## Beschreibung

Die Erfindung betrifft Verfahren zur Herstellung von 2-Chlor-5-chlormethyl-1,3-thiazol (CCT).

2-Chlor-5-chlormethyl-1,3-thiazol ist ein wertvolles Zwischenprodukt bei der Herstellung von Pestiziden oder von pharmazeutischen Produkten.

Aus der Literatur ist bereits eine Vielzahl der unterschiedlichsten Verfahren zur Herstellung von CCT bekannt.

So wird beispielsweise in EP 0 260 560 und in EP 0 446 913 die Herstellung von CCT durch Reaktion von Allylisothiocyanat bzw. von mit einer Abgangsgruppe substituierten Allylisothiocyanat mit einem Chlorierungsmittel und in EP 0 763 531 die Umsetzung von 2-Chlorallylisothiocyanat mit einem Chlorierungsmittel beschrieben. Diese Verfahren weisen Nachteile auf, da z.B. bei der ersten Variante mehrere Nebenprodukte auftreten, wodurch das hergestellte CCT eine geringe Reinheit aufweist, und das Ausgangsmaterial bei der zweiten Variante nur zu hohen Kosten erhältlich ist. Weiters muss ein beträchtlicher Überschuss an Chlorierungsmittel verwendet werden und es muss in großer Verdünnung gearbeitet werden. Ebenso ist ein exaktes Einhalten der Reaktionstemperatur erforderlich und die sich im Reaktionsverlauf bildende stabile Zwischenstufe muss in einem zusätzlichen Reaktionsschritt exotherm in das gewünschte Endprodukt überführt werden. Als Verbesserung wird in EP 0 794 180 die Herstellung von CCT aus 1,3-Dichlorpropen und einem Thiocyanatsalz über 3-Chlor-1-isothiocyanat-1-propen beschrieben.

Andere Varianten, wie etwa das Verfahren gemäß EP 0 775 700, wonach CCT über 2-Amino-5-methylthiazol mittels Diazotierung und anschließender Chlorierung hergestellt wird, zeigen ebenfalls den Nachteil, dass CCT durch eine Vielzahl von Nebenprodukten verunreinigt ist, die kaum bzw. nur sehr schwierig und unter hohen Ausbeuteverlusten abzutrennen sind.

Aufgabe der Erfindung war es ein neues Verfahren zu finden, das die Herstellung von CCT in hoher Reinheit und Ausbeute ermöglicht.

Gegenstand der Erfindung ist demnach ein Verfahren zur Herstellung von 2-Chlor-5-chlormethyl-1,3-thiazol, das dadurch gekennzeichnet ist, dass Allylisothiocyanat der Formel CH₂=CH-CH₂-NCS
a) bei -40°C bis +30°C in einem unter den Reaktionsbedingungen inerten Lösungsmittel mit 1 bis 2 mol eines Chlorierungsmittels pro mol Allylisothiocyanat umgesetzt wird und
b) das so erhaltene Reaktionsgemisch bei einer Reaktionstemperatur von 0°C bis zur Siedetemperatur des eingesetzten Lösungsmittels mit 1 bis 5 mol Oxidationsmittel ausgewählt aus der Gruppe von Br₂, N-Halogenimide, oder Dihalogendialkylhydantoine pro mol Allylisothiocyanat versetzt wird und
c) 2-Chlor-5-chlormethyl-1,3-thiazol aus dem Reaktionsgemisch isoliert wird und
d) gegebenenfalls durch Kristallisation in hochreines 2-Chlor-5-chlormethyl-1,3-thiazol überführt wird.

Als Ausgangsverbindung zur Herstellung von CCT wird erfindungsgemäß von Allylisothiocyanat der Formel CH₂=CH-CH₂-NCS ausgegangen.

Diese Verbindung wird in Schritt a) mit einem Chlorierungsmittel umgesetzt.

Als Chlorierungsmittel kommen dabei Chlor und Verbindungen in Frage, die unter den Reaktionsbedingungen Chlor abspalten. Dies sind beispielsweise Sulfurylchlorid, PCl₅, PCl₃, POCl₃ u.s.w.

Das Chlorierungsmittel wird erfindungsgemäß in einer Menge von 1 bis 2 mol pro mol Allylisothiocyanat eingesetzt. Bevorzugt werden 1 bis 1,6 und besonders bevorzugt 1 bis 1,3 mol Chlorierungsmittel pro mol Allylisothiocyanat verwendet.

Die Umsetzung erfolgt dabei in einem unter den Reaktionsbedingungen inerten Lösungsmittel. Geeignete Lösungsmittel sind beispielsweise aliphatische oder aromatische Kohlenwasserstoffe, wie etwa Benzol, Toluol, Hexan, Heptan, Octan u.s.w., halogenierte aliphatische oder aromatische Kohlenwasserstoffe, wie etwa Dichlormethan, 1,2-Dichlorethan, Tetrachlorkohlenstoff, 1,1,2,2,-Tetrachlorethan, Trichlormethan- und ethan, Chlorbenzol, Dichlorbenzole, Trichlorbenzol u.s.w., Ether, wie etwa Diethylether, Diisopropylether, Tetrahydrofuran, Dioxan, Dimethoxyethan, u.s.w., Nitrile, wie etwa Acetonitril, Propionitril, u.s.w., Amide, wie etwa Dimethylformamid, Methylpyrrolidon, Diethylformamid, u.s.w., Sulfoxide, wie etwa Dimethylsulfoxid u.s.w. Bevorzugt werden halogenierte, aliphatische oder aromatische Lösungsmittel aus der Gruppe Dichlormethan, 1,2-Dichlorethan, Tetrachlorkohlenstoff, 1,1,2,2,-Tetrachlorethan, Trichlormethan- und ethan, Chlorbenzol und Dichlorbenzole verwendet.

Die Reaktionstemperatur liegt zwischen -40°C und +30°C, bevorzugt zwischen -30°C und +10°C und besonders bevorzugt zwischen -20°C und 0 °C.

Das Reaktionsgemisch wird dabei für einige Minuten bis zu mehreren Stunden bei der entsprechenden Reaktionstemperatur gerührt. Bevorzugt wird für etwa 5 Minuten bis zu 5 Stunden und besonders bevorzugt für etwa 20 Minuten bis zu 2 Stunden gerührt.

Durch die Reaktion von Allylisothiocyanat und Chlorierungsmittel wird eine Zwischenverbindung der Formel 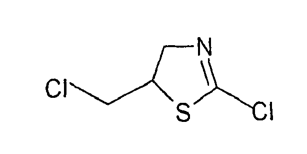 erhalten, die jedoch nicht aus dem Reaktionsgemisch isoliert wird.

Das durch Schritt a) erhaltene Reaktionsgemisch, das obige Zwischenverbindung enthält, wird sofort nach Schritt a) der nachfolgenden Umsetzung mit Oxidationsmittel (Schritt b) unterzogen.

Dazu wird das Reaktionsgemisch in Schritt b) met 1 bis 5 mol einem Oxidationsmittel, das gleichzeitig als Halogenierungsmittel fungiert, versetzt, wobei die Oxidation unter Halogenierung mit anschließender Dehydrohalogenierung erfolgt. Die Halogenierungsmittel sind chlorierende oder bromierende Verbindungen ausgewählt aus der Gruppe von Br₂, N-Halogenimide, oder Dihalogendialkylhydantoine, wie etwa Dichlordimethylhydantoin.

Bevorzugt werden 1,2 bis 4 mol und besonders bevorzugt 1,8 bis 3 mol Oxidationsmittel bzw. Halogenierungsmittel pro mol Allylisothiocyanat verwendet.

Bevorzugt werden als Oxidationsmittel bzw. Halogenierungsmittel N-Chlor- oder N-Bromsuccinimid, N-Chlor- oder N-Bromphthalimid und Dichlordimethylhydantoin eingesetzt.

Wird eine halogenierende Verbindung als Oxidationsmittel eingesetzt, so erfolgt eine Substitution, die beispielsweise durch UV-Licht und/oder durch Zugabe eines geeigneten Initiators gestartet oder beschleunigt wird. Geeignete Initiatoren sind übliche, aus dem Stand der Technik bekannte Verbindungen. Dies sind beispielsweise Peroxide, wie etwa Dibenzoylperoxid, Diacetylperoxid, Azoverbindungen, wie etwa Azobisisobutyronitril, u.s.w. Der Initiator wird dabei in einer Menge von 0,05 bis 10 mol%, bevorzugt von 0,1 bis 8 mol% und besonders bevorzugt von 0,5 bis 5 mol%, bezogen auf Allylisothiocyanat, eingesetzt.

Das Oxidationsmittel und/oder der Initiator kann dabei entweder in einer Portion oder aufgeteilt auf mehrere Portionen zugesetzt werden.

Die Reaktionstemperatur liegt zwischen 0°C und dem Siedepunkt des verwendeten Lösungsmittel. Bevorzugt liegt die Reaktionstemperatur zwischen 20°C und dem Siedepunkt des verwendeten Lösungsmittel und besonders bevorzugt zwischen etwa 30 und 80°C.

Von besonderen Vorteil für die Reinheit des CCT ist es, wenn die Umsetzungen gemäß Schritt a) und b) möglichst wasserfrei durchgeführt werden. Dies wird durch Verwendung absoluter Lösungsmittel und von reinem Allylisothiocyanat, sowie gegebenenfalls Arbeiten unter Inertgasatmosphäre erreicht.

Zur Isolierung und Aufarbeitung des hergestellten CCT wird das Reaktionsgemisch gegebenenfalls zuerst abgekühlt.

Wird ein N-Halogenimid als Oxidationsmittel eingesetzt, so wird ausgefallenes Imid beispielsweise gegebenenfalls durch Filtration abgetrennt. Anschließend wird das verbleibende Reaktionsgemisch basisch gestellt, um eventuell vorhandene Säuren, wie HCl oder HBr zu binden. Dies kann beispielsweise durch Waschen der Versetzen mit geeigneten Basen erfolgen. Geeignete Basen sind beispielsweise NaHCO₃-Lösungen, KHCO₃-Lösungen, Na₂CO₃-Lösungen, K₂CO₃-Lösungen, verdünnte NaOH oder KOH, Ammoniakwasser, trockenes Na₂CO₃oder K2CO₃ u.s.w. Nachfolgend wird das Lösungsmittel abgetrennt und das rohe CCT beispielsweise durch eine einfache Destillation gereinigt.

Zur weiteren Steigerung der Reinheit kann sodann das CCT-enthaltende Destillat einer Kristallisation und Waschen mit bzw. Digerieren in einem aliphatischen Kohlwasserstoff, wie etwa Hexan, Heptan u.s.w., in einem Ether oder Ester unterzogen werden. Bevorzugt sind aliphatische Kohlenwasserstoffe.

Um hochreines CCT zu erhalten wird demnach bevorzugt, gegebenenfalls nach Abtrennung von ausgefallenem Imid, basischem Waschen, Abtrennung des Lösungsmittels und Destillation, das Destillat durch Abkühlen auf 0 bis -40°C, bevorzugt auf -5 bis -40°C und besonders bevorzugt auf -15 bis -40°C auskristallisiert, die Kristalle abgesaugt und kalt in einem aliphatischen, bevorzugt eiskaltem, Kohlenwasserstoff, bevorzugt in Hexan oder Heptan, oder in einem Ether oder Ester digeriert und anschließend bei Raumtemperatur im Vakuum, gegebenenfalls unter Stickstoffatmosphäre, getrocknet.

Bei der Kristallisation enthält die über den Kristallen befindliche Flüssigkeit meist bis zu 29Gew.% CCT, d.h. ab CCT-Konzentrationen von über 30% kann hochreines Material aus solchen Rohprodukten gewonnen werden.

Durch die Kristallisation können jedoch nicht nur Destillate, sondern auch festes, im Handel erhältliches CCT weiter aufgereinigt werden. Dazu wird das zu reinigende CCT zuerst in einem aliphatischen Kohlenwasserstoff, einem Ether oder Ester aufgelöst, die Lösung gegebenenfalls mit Aktivkohle versetzt und gegebenenfalls vorhandene Feststoffe abfiltriert. Anschließend erfolgt analog obiger Beschreibung die Kristallisation mit nachfolgendem Digerieren.

Durch die erfindungsgemäße Reaktionsführung wird CCT in, im Vergleich zum Stand der Technik, wesentlich höheren Reinheiten erhalten. Wie Vergleichsversuche zeigen, weist ein erfindungsgemäß hergestelltes Roh-CCT kaum hochmolekulare Nebenprodukte auf, wie dies beispielsweise bei CCT, hergestellt gemäß EP 0 260 560, der Fall ist.

Diese hochmolekularen Nebenprodukte sind mittels GC nicht oder nur äußerst schwierig zu erkennen, mittels HPLC-MS können sie aber nachgewiesen werden. Durch die erfindungsgemäße Aufarbeitung und Reinigung wird CCT in, im Vergleich zum Stand der Technik, wesentlich höheren Reinheiten erhalten.

Ein weiterer Vorteil des erfindungsgemäßen Verfahrens ist die Durchführung als Eintopfreaktion, die keine aufwendigen Apparate benötigt.

### Beispiel 1

20g (0,2 Mol) destilliertes Allylisothiocyanat wurden in 200ml Dichlormethan bei -10 bis - 15°C mit 29,7g (0,22 Mol) Sulfurylchlorid versetzt und eine Stunde bei -15°C gerührt. Dann wurden 53,36g (0,4 Mol) N-Chlorsuccinimid und 0,65g (4mMol) Azo-bis-isobutyronitril zugegeben und unter UV-Licht 3 Stunden zum Sieden erhitzt.

Das Reaktionsgemisch wurde gekühlt, ausgefallenes Succinimid abfiltriert und der Niederschlag 2mal mit je 5-10ml gekühltem Dichlormethan gewaschen.

Die Filtrate wurden zwei mal mit je 50ml 5%iger NaHCO₃-Lösung gewaschen, über Natriumsulfat getrocknet und das Lösungsmittel abgezogen.
Ausbeute analytisch: 22,18g CCT (entspricht 66% d. Th.)

### (Die quantitative gaschromatographische Analyse erfolgte stets mit Tetradecan als internem Standard bzw. einer abgeleiteten Methode mit externem Standard.)

Das Rohprodukt wurde durch Destillation bei 60-65°C / 2mbar gereinigt. Es wurde ein Destillat mit einem CCT-Gehalt von 85% erhalten.
Ausbeute analytisch: 17,75g CCT (52% d.Th.)

Das destillierte Rohprodukt wurde durch Kühlen auf -20°C zur Kristallisation gebracht, die Kristalle wurden abgesaugt, 2 mal mit je 5ml kaltem Hexan digeriert und bei Raumtemperatur im Vakuum getrocknet.
Ausbeute: 13,8g farblose Kristalle mit CCT-Gehalt von 99,8% (41 % d. Th.)

### Beispiel 2

4,9g (50mMol) destilliertes Allylisothiocyanat wurden in 60ml Dichlormethan bei -10°C innerhalb von 30 Minuten mit 6,7g (50mMol) Sulfurylchlorid versetzt und eine Stunde bei -10°C gerührt.

Dann wurden 13,3g N-Chlorsuccinimid zugegeben und zum Sieden erhitzt. Anschließend wurden portionsweise innerhalb von 3 Stunden insgesamt 240mg (1,5mMol) Azo-bis-isobutyronitril in 4 ml Dichlormethan zugegeben und eine weitere halbe Stunde erhitzt. Das Reaktionsgemisch wurde mit 25ml Natriumhydrogencarbonat-Lösung und 20ml Wasser gewaschen, über Natriumsulfat getrocknet und filtriert.
Ausbeute analytisch: 4,6g CCT (55% d.Th.)

Das Rohprodukt wurde destillativ bei 103-110°C / 17mbar gereinigt. Das Destillat enthielt 74 Gew% CCT.
Ausbeute analytisch: 4,0g CCT (48% d.Th.)

### Beispiel 3

19,6g (0,2 Mol) destilliertes Allylisothiocyanat wurden in 240ml Dichlormethan bei -10°C innerhalb von 75 Minuten mit 26,8 (0,2 Mol) Sulfurylchlorid versetzt und eine Stunde bei - 10°C gerührt.

Dann wurden 26,6g (0,2 Mol) N-Chlorsuccinimid und 0,32g (2mMol) Azo-bis-isobutyronitril in 8ml Dichlormethan zugegeben und zum Sieden erhitzt. Danach wurden drei mal im stündlichen Abstand jeweils 13,3g (0,1 Mol) N-Chlorsuccinimid und 0,32g (2mMol) Azo-bis-isobutyronitril in 8ml Dichlormethan zugegeben und zuletzt noch 2 Stunden zum Sieden erhitzt.
Ausbeute analytisch: 21,7g CCT (64,5% d.Th.)

Das Reaktionsgemisch wurde mit 150ml gesättigter Natriumhydrogencarbonatlösung und drei mal mit je 100ml Wasser gewaschen, über Natriumsulfat getrocknet und das Lösungsmittel abgezogen.
Ausbeute analytisch: 18,8g CCT (56% d.Th.)

### Beispiel 4

9,9g (0,1 Mol) destilliertes Allylisothiocyanat wurden in 100ml Dichlorethan bei -10°C innerhalb von 45 Minuten mit 14,8 (0,11 Mol) Sulfurylchlorid versetzt und eine Stunde bei - 10°C gerührt.

Dann wurden 29,3g (0,22 Mol) N-Chlorsuccinimid und 0,32g (2mMol) Azo-bis-isobutyronitril in 5ml Dichlorethan zugegeben und 2 Stunden auf 70°C erhitzt.

Das Reaktionsgemisch wurde auf -5°C abgekühlt und das ausgefallene Succinimid abfiltriert.
Ausbeute analytisch: 9,46g CCT (56,3% d.Th.)

### Beispiel 5

24,8g (0,25 Mol) destilliertes Allylisothiocyanat wurden in 100ml Dichlorethan bei -10°C innerhalb von 90 Minuten mit 37,1 g (0,27 Mol) Sulfurylchlorid versetzt und eine Stunde bei -10°C gerührt.

Dann wurden 33,4g (0,25 Mol) N-Chlorsuccinimid und 0,82g (5mMol) Azo-bis-isobutyronitril zugegeben und auf 70°C erhitzt. Nach ½ Stunde und nach 1 Stunde wurden jeweils weitere 20,0g (0,15 Mol) N-Chlorsuccinimid und 0,41g (2,5mMol) Azo-bis-isobutyronitril zugegeben und zuletzt noch 2 Stunden bei 70°C gerührt.

Das Reaktionsgemisch wurde auf Raumtemperatur abgekühlt und das ausgefallene Succinimid abfiltriert.
Ausbeute analytisch: 16,8g CCT (40% d.Th.)

### Beispiel 6

31,45g (0,315 Mol) Allylisothiocyanat wurden in 100ml Dichlormethan bei -15 bis -10°C innerhalb von 2 Stunden mit 44,67g (0,331 Mol) Sulfurylchlorid versetzt und eine Stunde bei -10°C gerührt.

Dann wurden 42,02g (0,315 Mol) N-Chlorsuccinimid zugegeben und unter UV-Licht zum Sieden erhitzt. Nach ½ Stunde und nach einer weiteren Stunde wurden jeweils weitere 25,35g (0,19 Mol) N-Chlorsuccinimid und zuletzt noch 2 ½ Stunden unter UV-Licht erhitzt. Das Reaktionsgemisch wurde auf Raumtemperatur abgekühlt und das ausgefallene Succinimid abfiltriert.
Ausbeute analytisch: 25,4g CCT (48% d.Th.)

Die Rohprodukt-Lösung wurde mit 50,0g (0,47 Mol) Natriumcarbonat versetzt, ½ Stunde bei Raumtemperatur gerührt, filtriert und das Lösungsmittel abgezogen.
Ausbeute analytisch: 24,94g CCT (47% d.Th.)

Das Rohprodukt wurde durch Destillation bei 66-70°C / 3mbar gereinigt, es wurden Destillate mit CCT-Gehalt von 77 Gew.% erhalten.
Ausbeute analytisch: 36% d.Th.

### Beispiel 7

In eine Lösung von 10,91 g (0,11 Mol) Allylisothiocyanat in 100ml Dichlormethan wurden bei -15 bis -10°C innerhalb von 1 Stunde 7,8g (0,11 Mol) Chlor eingeleitet und eine Stunde bei -10°C gerührt.

Dann wurden 22,03g (0,165 Mol) N-Chlorsuccinimid und 0,36g (2,2mMol) Azo-bis-isobutyronitril zugegeben und zum Sieden erhitzt. Danach wurden fünf mal im Abstand von je ½ Stunde jeweils 1,47g (0,11 mMol) N-Chlorsuccinimid und 70mg (0,44 mMol) Azo-bis-isobutyronitril zugegeben und danach 10 Stunden zum Sieden erhitzt.

Das Reaktionsgemisch wurde auf 5°C abgekühlt und das ausgefallene Succinimid abfiltriert.
Ausbeute analytisch: 10,54g CCT (57% d.Th.)

### Vergleichsbeispiel 1 (analog EP 0 260 560):

75ml Chloroform wurden bei Siedetemperatur mit Chlor gesättigt. In die Lösung wurden bei Siedetemperatur innerhalb von 2 Stunden 25g (250mMol) Allylisothiocyanat in 50ml Chloroform zugepumpt und gleichzeitig 133,7g (1,91Mol) Chlorgas eingeleitet, wobei ständig überschüssiges Chlorgas vorhanden war. Danach wurden innerhalb einer ½ Stunde weiterhin bei Siedetemperatur zusätzlich 17,5g (0,25Mol) Chlor eingeleitet. Überschüssiges Chlor wurde mit einem Stickstoffstrom ausgetrieben und die Ausbeute mittels GC-Analyse bestimmt. Die erhaltene Lösung (199,29g) enthielt 6,99Gew% bzw. 43Fl% CCT. Ausbeute analytisch: 13,93g CCT (33% d.Th.)

### Vergleichsbeispiel II (analog EP 0 260 560):

75ml Dichlormethan wurden bei Siedetemperatur mit Chlor gesättigt. In die Lösung wurden bei Siedetemperatur innerhalb von 1 Stunde 25g (250mMol) Allylisothiocyanat in 50ml Dichlormethan zugepumpt und gleichzeitig 100g (1,43Mol) Chlorgas eingeleitet, wobei ständig überschüssiges Chlorgas vorhanden war. Danach wurden innerhalb einer ½ Stunde weiterhin bei Siedetemperatur zusätzlich 85g (1,21 Mol) Chlor eingeleitet. Überschüssiges Chlor wurde mit einem Stickstoffstrom ausgetrieben und die Ausbeute mittels GC-Analyse bestimmt. Die erhaltene Lösung (80,02g) enthielt 15,43Gew% bzw. 38,8Fl% CCT. Ausbeute analytisch: 12,35g CCT (29% d.Th.)

### Beispiel 8:

33,56g eines Rohdestillates, hergestellt analog Beispiel 2, mit CCT-Gehalt von 70 Gew% wurde bei -20°C durch Zusatz von Impfkristallen zur Kristallisation gebracht. Die Kristalle wurden abgesaugt und zwei mal mit insgesamt 25ml eiskaltem Hexan digeriert.
Ausbeute: 15,08g farblose Kristalle; Qualität: 99,6 Gew% CCT
=64% des eingesetzten CCT (Mutterlauge enthält noch 43% CCT)

### Beispiel 9:

12,20g eines Rohdestillats, welches 43,6 Gew.% CCT enthielt, wurden durch Zusatz von Impfkristallen bei -20°C zur Kristallisation gebracht.

Die Kristalle wurden abgesaugt und mit 2ml eisgekühltem Hexan nachgewaschen.
Ausbeute: 2,23g farblose Kristalle; Qualität: 99,2 Gew% CCT
=42% des eingesetzten CCT (Mutterlauge enthält 27% CCT)

### Beispiel 10:

10,0g CCT (98,2Gew% CCT, Fa. Fine Organics) wurden bei Raumtemperatur in 30ml Hexan gelöst, mit 0,5g Aktivkohle versetzt, 15 Minuten gerührt und anschließend filtriert. Es wurde 2 mal mit insgesamt 4ml Hexan nachgewaschen und das Filtrat auf -20°C gekühlt. Die ausgefallenen Kristalle wurden abfiltriert, kalt mit 5ml Hexan digeriert und im Vakuum bei Raumtemperatur getrocknet.
Ausbeute: 6,74g farblose Kristalle; Qualität: 101,3 Gew% CCT

### Beispiel 11:

10,0g CCT (98,2Gew% CCT) wurden bei 50°C in 16ml Hexan gelöst, danach 15 Minuten bei Raumtemperatur gerührt, anschließend filtriert und das Filtrat auf -20°C gekühlt. Die ausgefallenen Kristalle wurden abfiltriert, kalt mit 2ml Hexan digeriert und im Vakuum bei Raumtemperatur getrocknet.
Ausbeute: 9,06g farblose Kristalle; Qualität: 100,2 Gew% CCT

## Patentansprüche

1. Verfahren zur Herstellung von 2-Chlor-5-chlormethyl-1,3-thiazol, das **dadurch gekennzeichnet ist, dass** Allylisothiocyanat der Formel CH₂=CH-CH₂-NCS
a) bei -40°C bis +30°C in einem unter den Reaktionsbedingungen inerten Lösungsmittel mit 1 bis 2 mol eines Chlorierungsmittels pro mol Allylisothiocyanat umgesetzt wird
und
b) das so erhaltene Reaktionsgemisch bei einer Reaktionstemperatur von 0°C bis zur Siedetemperatur des eingesetzten Lösungsmittels mit 1 bis 5 mol Oxidationsmittel ausgewählt aus der Gruppe von Br₂, N-Halogenimide, und Dihalogendialkylhydantoine pro mol Allylisothiocyanat versetzt wird und
c) 2-Chlor-5-chlormethyl-1,3-thiazol aus dem Reaktionsgemisch isoliert wird und
d) gegebenenfalls durch Kristallisation in hochreines 2-Chlor-5-chlormethyl-1,3-thiazol überführt wird.

2. Verfahren nach Anspruch 1, **dadurch gekennzeichnet, dass** als inertes Lösungsmittel ein aliphatischer oder aromatischer Kohlenwasserstoff aus der Gruppe Benzol, Toluol, Hexan, Heptan, Octan, ein halogenierter, aliphatischer oder aromatischer Kohlenwasserstoff aus der Gruppe Dichlormethan, 1,2-Dichlorethan, Tetrachlorkohlenstoff, 1,1,2,2,-Tetrachlorethan, Trichlormethan- und ethan, Chlorbenzol, Dichlorbenzole, Trichlorbenzol, ein Ether aus der Gruppe Diethylether, Diisopropylether, Tetrahydrofuran, Dioxan, Dimethoxyethan, ein Nitril aus der Gruppe Acetonitril, Propionitril, ein Amid aus der Gruppe Dimethylformamid, Methylpyrrolidon, Diethylformamid oder ein Sulfoxid eingesetzt wird.

3. Verfahren nach Anspruch 1, **dadurch gekennzeichnet, dass** in Schritt a) als Chlorierungsmittel Chlor oder eine chlorabspaltende Verbindung aus der Gruppe Sulfurylchlorid, PCl₅, PCl₃ oder POCl₃ eingesetzt wird.

4. Verfahren nach Anspruch 1, **dadurch gekennzeichnet, dass** als N-Halogenimide N-Chlorsuccinimid, N-Bromsuccinimid, N-Chlorphthalimid oder N-Bromphthalimid und als Dihalogendialkylhydantoine Dichlordimethylhydantoin verwendet werden.

5. Verfahren nach Anspruch 1, **dadurch gekennzeichnet, dass** in Schritt b) bei Verwendung von halogenierenden Verbindungen als Oxidationsmittel die Reaktion mittels UV-Licht und/oder durch Zugabe eines Initiators gestartet oder beschleunigt wird.

6. Verfahren nach Anspruch 1, **dadurch gekennzeichnet, dass** Schritt a) und Schritt b) wasserfrei, unter Verwendung von absoluten Lösungsmittel und gegebenenfalls unter Verwendung einer Inertgasatmosphäre, durchgeführt werden.

7. Verfahren nach Anspruch 1, **dadurch gekennzeichnet, dass** zur Isolierung des 2-Chlor-5-chlormethyl-1,3-thiazols, das nach Schritt b) erhaltene Reaktionsgemisch gegebenenfalls zuerst abgekühlt, gegebenenfalls ausgefallene Verbindungen abgetrennt werden, worauf das verbleibende Reaktionsgemisch mit einer Base gewaschen oder versetzt wird, Lösungsmittel abgetrennt und 2-Chlor-5-chlormethyl-1,3-thiazol durch Destillation erhalten wird.

8. Verfahren nach Anspruch 1, **dadurch gekennzeichnet, dass** in Schritt d) zur Herstellung von hochreinem 2-Chlor-5-chlormethyl-1,3-thiazol das in Schritt c) erhaltene Destillat durch Abkühlen auf 0 bis -40°C auskristallisiert wird, die Kristalle abgesaugt und kalt in einem aliphatischen Kohlenwasserstoff, einem Ether oder einem Ester digeriert und anschließend getrocknet werden, oder dass festes 2-Chlor-5-chlormethyl-1,3-thiazol zuerst in einem aliphatischen Kohlenwasserstoff, einem Ether oder einem Ester gelöst wird, die Lösung gegebenenfalls mit Aktivkohle versetzt wird und gegebenenfalls vorhandene Feststoffe abgetrennt werden, worauf durch Abkühlen auf 0 bis -40°C auskristallisiert wird, die Kristalle abgesaugt und kalt in einem aliphatischen Kohlenwasserstoff, einem Ether oder einem Ester digeriert und anschließend getrocknet werden.

## Claims

1. Process for preparing 2-chloro-5-chloromethyl-1,3-thiazole, **characterized in that** allyl isothiocyanate of the formula CH₂=CH-CH₂-NCS
a) is reacted at -40°C to +30°C in a solvent which is inert under the reaction conditions with 1 to 2 mol of a chlorinating agent per mole of allyl isothiocyanate and
b) the reaction mixture thus obtained is admixed at a reaction temperature of 0°C up to the boiling temperature of the solvent used with 1 to 5 mol of oxidizing agent selected from the group of Br₂, N-haloimides and dihalodialkylhydantoins per mole of allyl isothiocyanate, and
c) 2-chloro-5-chloromethyl-1,3-thiazole is isolated from the reaction mixture and
d) optionally converted by crystallization to high-purity 2-chloro-5-chloromethyl-1,3-thiazole.

2. Process according to Claim 1, **characterized in that** the inert solvent used is an aliphatic or aromatic hydrocarbon from the group of benzene, toluene, hexane, heptane, octane, a halogenated aliphatic or aromatic hydrocarbon from the group of dichloromethane, 1,2-dichloroethane, carbon tetrachloride, 1,1,2,2-tetrachloroethane, trichloromethane and trichloroethane, chlorobenzene, dichlorobenzenes, trichlorobenzene, an ether from the group of diethyl ether, diisopropyl ether, tetrahydrofuran, dioxane, dimethoxyethane, a nitrile from the group of acetonitrile, propionitrile, an amide from the group of dimethylformamide, methylpyrrolidone, diethylformamide or a sulphoxide.

3. Process according to Claim 1, **characterized in that** the chlorinating agent used in step a) is chlorine or a chlorine-releasing compound from the group of sulfuryl chloride, PCl₅, PCl₃ or POCl₃.

4. Process according to Claim 1, **characterized in that** the N-haloimides used are N-chlorosuccinimide, N-bromosuccinimide, N-chlorophthalimide or N-bromophthalimide, and the dihalodialkylhydantoin used is dichlorodimethylhydantoin.

5. Process according to Claim 1, **characterized in that** the reaction in step b), in the case of use of halogenating compounds as the oxidizing agent, is initiated or accelerated by means of UV light and/or by addition of an initiator.

6. Process according to Claim 1, **characterized in that** step a) and step b) are performed under anhydrous conditions using absolute solvent and optionally using an inert gas atmosphere.

7. Process according to Claim 1, **characterized in that** t h e 2-chloro-5-chloromethyl-1,3-thiazole is isolated by optionally first cooling the reaction mixture obtained after step b), removing any precipitated compounds, and then washing or admixing the remaining reaction mixture with a base, removing solvent, and obtaining 2-chloro-5-chloromethyl-1,3-thiazole by distillation.

8. Process according to Claim 1, **characterized in that** high-purity 2-chloro-5-chloromethyl-1,3-thiazole is prepared in step d) by crystallizing the distillate obtained in step c) by cooling to 0 to -40°C, filtering off the crystals with suction and digesting them cold in an aliphatic hydrocarbon, an ether or an ester, and then drying, or by first dissolving solid 2-chloro-5-chloromethyl-1,3-thiazole in an aliphatic hydrocarbon, an ether or an ester, optionally admixing the solution with activated carbon and optionally removing solids present, and then crystallizing by cooling to 0 to -40°C, filtering the crystals off with suction and digesting them cold in an aliphatic hydrocarbon, an ether or an ester, and then drying.

## Revendications

1. Procédé pour la préparation de 2-chloro-5-chlorométhyl-1,3-thiazole, qui est **caractérisé en ce qu'**on fait réagir de l'isothiocyanate d'allyle de formule CH₂=CH-CH₂-NCS
a) à une température de -40 °C à +30 °C, dans un solvant inerte dans les conditions réactionnelles, avec 1 à 2 moles d'un agent de chloration par mole d'isothiocyanate d'allyle et
b) on ajoute au mélange réactionnel ainsi obtenu, à une température de réaction dans la plage allant de 0 °C à la température d'ébullition du solvant utilisé, avec 1 à 5 moles d'un oxydant choisi dans le groupe constitué par Br₂, des N-halogénimides, et des dihalogénodialkylhydantoïnes, par mole d'isothiocyanate d'allyle et
c) on isole le 2-chloro-5-chlorométhyl-1,3-thiazole à partir du mélange réactionnel et
d) éventuellement on le convertit par cristallisation en 2-chloro-5-chlorométhyl-1,3-thiazole de grande pureté.

2. Procédé selon la revendication 1, **caractérisé en ce qu'**on utilise comme solvant inerte un hydrocarbure aliphatique ou aromatique choisi dans le groupe constitué par le benzène, le toluène, l'hexane, l'heptane, l'octane, un hydrocarbure aliphatique ou aromatique halogéné choisi dans le groupe constitué par le dichlorométhane, le 1,2-dichloréthane, le tétrachlorure de carbone, le 1,1,2,2-tétrachloréthane, le trichlorométhane et le trichloréthane, le chlorobenzène, les dichlorobenzènes, le trichlorobenzène, un éther choisi dans le groupe constitué par l'oxyde de diéthyle, l'éther diisopropylique, le tétrahydrofuranne, le dioxanne, le diméthoxyéthane, un nitrile choisi dans le groupe constitué par l'acétonitrile, le propionitrile, un amide choisi dans le groupe constitué par le diméthylformamide, la méthylpyrrolidone, le diéthylformamide ou un sulfoxyde.

3. Procédé selon la revendication 1, **caractérisé en ce que** dans l'étape a) on utilise comme agent de chloration le chlore ou un composé libérant du chlore, choisi dans le groupe constitué par le chlorure de sulfuryle, PCl₅, PCl₃ ou POCl₃.

4. Procédé selon la revendication 1, **caractérisé en ce qu'**on utilise comme N-halogénimides le N-chlorosuccinimide, le N-bromosuccinimide, le N-chlorophtalimide ou le N-bromophtalimide et comme dihalogénodiakylhydantoïnes la dichlorodiméthylhydantoïne.

5. Procédé selon la revendication 1, **caractérisé en ce que** dans l'étape b) lorsqu'on utilise comme oxydant des composés halogénant on déclenche ou accélère la réaction à l'aide de lumière UV et/ou par addition d'un amorceur.

6. Procédé selon la revendication 1, **caractérisé en ce qu'**on effectue l'étape a) et l'étape b) en absence d'eau, avec utilisation de solvants anhydres et éventuellement avec utilisation d'une atmosphère de gaz inerte.

7. Procédé selon la revendication 1, **caractérisé en ce que** pour l'isolement du 2-chloro-5-chlorométhyl-1,3-thiazole éventuellement d'abord on refroidit le mélange réactionnel obtenu après l'étape b), éventuellement on sépare les composés précipités, à la suite de quoi le mélange réactionnel restant est lavé ou mélangé avec une base, on élimine le solvant et on obtient par distillation le 2-chloro-5-chlorométhyl-1,3-thiazole.

8. Procédé selon la revendication 1, **caractérisé en ce que** dans l'étape d) pour la préparation de 2-chloro-5-chlorométhyl-1,3-thiazole de grande pureté on fait cristalliser par refroidissement jusqu'à une température de 0 à -40 °C le distillat obtenu dans l'étape c), les cristaux sont séparés par filtration à la trompe et mis à digérer à froid dans un hydrocarbure aliphatique, un éther ou un ester et ensuite séchés, ou on dissout d'abord le 2-chloro-5-chlorométhyl-1,3-thiazole solide dans un hydrocarbure aliphatique, un éther ou un ester, éventuellement on ajoute du charbon actif à la solution et on sépare les solides éventuellement présents, puis on fait cristalliser par refroidissement jusqu'à une température de 0 à -40 °C, les cristaux sont séparés par filtration à la trompe et mis à digérer à froid dans un hydrocarbure aliphatique, un éther ou un ester et ensuite séchés.
